# EUROPEAN PATENT APPLICATION

(11) **EP 3 279 472 A2**
(43) Date of publication of application: **07.02.2018**
(21) Application number: 17180884.3
(22) Date of filing: 12.07.2017
(51) Int. Cl.: F04B 9/04, F04B 43/02, F04B 43/073

(54) **DIAPHRAGM PUMPS FOR MEDICAL APPLICATIONS**

(30) Priority: 13.07.2016 US 201615208726
(71) Applicant: Biosense Webster (Israel), Ltd., Yokneam, 2066717 (IL)
(72) Inventor: GOVARI, Assaf, 2066717 Yokneam (IL); ALGAWI, Yehuda, 2066717 Yokneam (IL)
(74) Representative: Carpmaels & Ransford LLP

(57) **Abstract**

Apparatus and methods described include a pump, which includes a piston, a diaphragm coupled to the piston and defining a wall of a fluid chamber, and a snail cam. The snail cam is configured to, by rotating, alternatingly (i) pump fluid from the fluid chamber, and (ii) draw fluid into the fluid chamber, by driving the piston to move the diaphragm. Other embodiments are also described.

## Description

### FIELD OF THE INVENTION

The present invention relates to the pumping of fluid, such as in the context of medical applications.

### BACKGROUND

US Patent 2,715,610, whose disclosure is incorporated herein by reference, describes a proportioner for chemical feed.

US Patent 8,590,810, whose disclosure is incorporated herein by reference, describes an air spray gun for paint. The air spray gun has a tubular body, having an integral screw thread receiving a threaded collar. This secures an air cap against the end of the tube. The air cap has four horns, formed with obliquely inwards directed bores, for patterning the spray, by impinging on it from opposite sides in a manner analogous to that for known air caps having two horns. Behind the air cap in the tube is an air distributor for distributing air to either or both of the pairs of air horns as required to patterning of the paint spray. Paint flow is controlled by a needle withdrawable from a paint nozzle for paint flow. The needle is in two parts, a front interchangeable part and a rear part connected to the trigger.

European Patent Publication EP0119210, whose disclosure is incorporated herein by reference, describes methods and means wherein a downhole reciprocating oil well pump powered by a source of pressurized fluid via one tubing string pumps liquid from the well through another tubing string. The downhole pump having internal means to vent gas and vapor from the pump chamber and to cause a pump stroke only when the pump chamber becomes filled with liquid. The pump chamber is sensed to be filled with liquid by a flow sensitive flow restrictor which monitors the venting of the gas and vapor and causes the vent to close when liquid flows past the restrictor thereby closing a power valve to allow the flow of power fluid towards the pump plunger. The plunger has a dome shaped head and the cylinder has ports to expel sediment from the pump chamber. The pump stroke speed may be independently regulated by adjusting the output of source of pressurized fluid and the return stroke may be adjusted by proper sizing of ports for return flow.

European Patent Publication EP0629777, whose disclosure is incorporated herein by reference, describes a fuel injection system for an internal combustion engine having improved fuel metering characteristics by means of minimizing any pressure pulses and fluctuations using a high pressure fuel pump having a plurality of positive displacement pumping devices, that are operating such that their delivery cycles overlap and such that the instantaneous speed of the pumping devices during their deliveries strokes is constant, so as to minimize pressure variations in the system and to avoid the necessity of having the pump being driven in a synchronized relationship to the engine output shaft. This permits the use of a variable speed drive so that the pump can be driven at speed ratios depending upon engine demand and/or eliminate the necessity for positive drives to maintain synchronization.

### SUMMARY OF THE INVENTION

An embodiment of the present invention provides a pump, including:
a piston;
a diaphragm coupled to the piston and defining a wall of a fluid chamber; and
a snail cam configured to, by rotating, alternatingly (i) pump fluid from the fluid chamber, and (ii) draw fluid into the fluid chamber, by driving the piston to move the diaphragm.

In a disclosed embodiment the snail cam is configured to, when rotating at a constant speed, alternatingly (i) pump fluid from the fluid chamber at a first rate, and (ii) draw fluid into the fluid chamber at a second rate that is greater than the first rate.

Alternatively, the snail cam is configured to, when rotating at a constant speed, during each rotational cycle of the snail cam, (i) pump fluid from the fluid chamber for a first amount of time, and (ii) draw fluid into the fluid chamber for a second amount of time that is less than the first amount of time. Typically, a ratio of the first amount of time to the second amount of time is at least 4:1.

In a further disclosed embodiment the snail cam is shaped to define a tooth having a slope of between 50 and 80 degrees. Alternatively or additionally, the snail cam is shaped to define a plurality of teeth.

In a yet further disclosed embodiment, over a majority of a circumference of the snail cam, a rate of increase of a radius of the snail cam is constant.

There is further provided, according to an embodiment of the present invention, apparatus, including:
a piston;
a diaphragm coupled to the piston and defining a wall of a fluid chamber;
a cam, configured to, by rotating, alternatingly (i) pump fluid from the fluid chamber, and (ii) draw fluid into the fluid chamber, by driving the piston to move the diaphragm;
a sensor, configured to sense a state of the cam and generate a sensor signal in response thereto; and
a processor, configured to control a rotating speed of the cam in response to the sensor signal.

Typically, the sensor consists of an optical sensor configured to capture an image of the cam, the sensor signal including the image. The apparatus may include a plurality of optical markers on the cam, the processor being configured to control the rotating speed of the cam in response to detecting the optical markers in the image.

The processor is typically configured to control the rotating speed of the cam such that the cam alternatingly (i) pumps fluid from the fluid chamber at a first rate, and (ii) draws fluid into the fluid chamber at a second rate that is greater than the first rate.

There is further provided a method, including:
providing (i) a piston, and (ii) a diaphragm coupled to the piston and defining a wall of a fluid chamber; and
by rotating a cam that drives the piston to move the diaphragm, alternatingly (i) pumping fluid from the fluid chamber, into a body of a subject, at a first rate, and (ii) drawing fluid into the fluid chamber at a second rate that is greater than the first rate.

Pumping the fluid from the fluid chamber may include pumping the fluid for at least 80% of a duration of each rotational cycle of the cam.

In an alternative embodiment pumping the fluid includes pumping the fluid into a heart of the subject.

In a further alternative embodiment pumping the fluid includes pumping the fluid at a constant rate.

The cam may be a snail cam.

The method may include controlling a rotating speed of the cam such that the second rate is greater than the first rate. Typically, controlling the rotating speed of the cam includes:
using a sensor, sensing a state of the cam and generating a sensor signal in response thereto; and
using a processor, controlling the rotating speed of the cam in response to the sensor signal.

The sensor may include an optical sensor, and the sensor signal may include an image captured by the optical sensor.

Controlling the rotating speed of the cam may consist of controlling the rotating speed of the cam in response to detecting, in the image, optical markers on the cam.

The present invention will be more fully understood from the following detailed description of embodiments thereof, taken together with the drawings, in which:

### BRIEF DESCRIPTION OF THE DRAWINGS

Fig. 1 is a schematic illustration of a system for cardiac ablation treatment, in accordance with some embodiments of the present invention;
Figs. 2A-B are schematic illustrations of an irrigation pump, in accordance with some embodiments of the present invention; and
Fig. 3 is a schematic illustration of apparatus for pumping fluid, in accordance with some embodiments of the present invention.

### DETAILED DESCRIPTION OF EMBODIMENTS

### OVERVIEW

In some medical applications, fluid is pumped into the body of a subject. For example, during some cardiac procedures, such as ablation procedures, an irrigating fluid, such as a saline fluid, is pumped into the subject's heart.

The inventors have observed that a peristaltic pump, which pumps fluid by compressing a tube that holds the fluid, has certain disadvantages. In particular, the peristaltic action of the pump introduces electrical noise, which can interfere with measurements of the heart's electrical activity. Furthermore, in some cases, the tube may begin to disintegrate, and the pump may consequently introduce small particles of tube-wall material (e.g., plastic) into the body of the patient.

Embodiments of the present invention, on the other hand, provide diaphragm pumps, which do not have the above-noted disadvantages. Each of the pumps described herein comprises a fluid chamber, a diaphragm that defines a wall of the fluid chamber, a piston that is coupled to the diaphragm, and a cam that, by rotating, drives the piston back and forth, thus alternatingly pumping fluid from, and drawing fluid into, the fluid chamber.

In some embodiments, a snail cam is used to drive the piston. Such a cam has a radius that increases over a large majority of the circumference of the cam, and then decreases sharply between the point of maximum radius and the point of minimum radius. Thus, for the vast majority of the rotational cycle of the cam, fluid is pumped from the fluid chamber, with only a relatively brief interruption for refilling of the fluid chamber. The nearly uninterrupted flow of fluid contributes to the safety and/or efficacy of the procedure.

Alternatively or additionally, the nearly uninterrupted flow may be accomplished by varying the rotational speed of the cam over each rotational cycle. That is, the cam may be rotated at a lower speed while pumping the fluid, and at a higher speed while drawing fluid into the fluid chamber. In some embodiments, a sensor, such as an optical sensor, senses the position of the piston, and a processor varies the speed of the cam in response to the position of the piston that is sensed by the sensor.

### SYSTEM DESCRIPTION

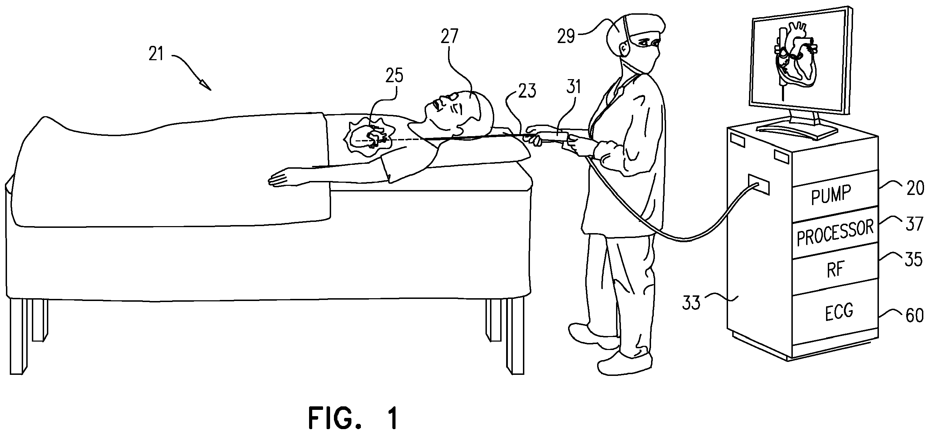

Reference is initially made to Fig. 1, which is a schematic illustration of a system 21 for cardiac ablation treatment, in accordance with an embodiment of the present invention. An operator 29 (such as an interventional cardiologist) inserts an intra-body probe, such as a catheter 23, via the vascular system of a patient 27, into a chamber of the patient's heart 25. For example, to treat atrial fibrillation, the operator may advance the catheter into the left atrium and bring an ablation electrode at a distal end of the catheter into contact with myocardial tissue that is to be monitored and/or ablated.

Catheter 23 is connected at its proximal end to a handle 31, which is connected, in turn, to a console 33. Console 33 comprises a radiofrequency (RF) generator 35, which supplies electrical power to the ablation electrode in order to ablate the target tissue. An irrigation pump 20 pumps an irrigating fluid, such as a saline solution, from a fluid reservoir (not shown), through catheter 23, and to the ablation electrode. The irrigating fluid is then passed into the heart during the ablation procedure, in order to help cool and/or dilute the blood.

During the procedure, a processor 37 may be used to monitor the ablating current and/or control the current by controlling RF energy generator 35, either automatically or in response to inputs from operator 29. Before, during, and/or after the procedure, an electrocardiogram (ECG) recorder 60 may record an ECG of the patient.

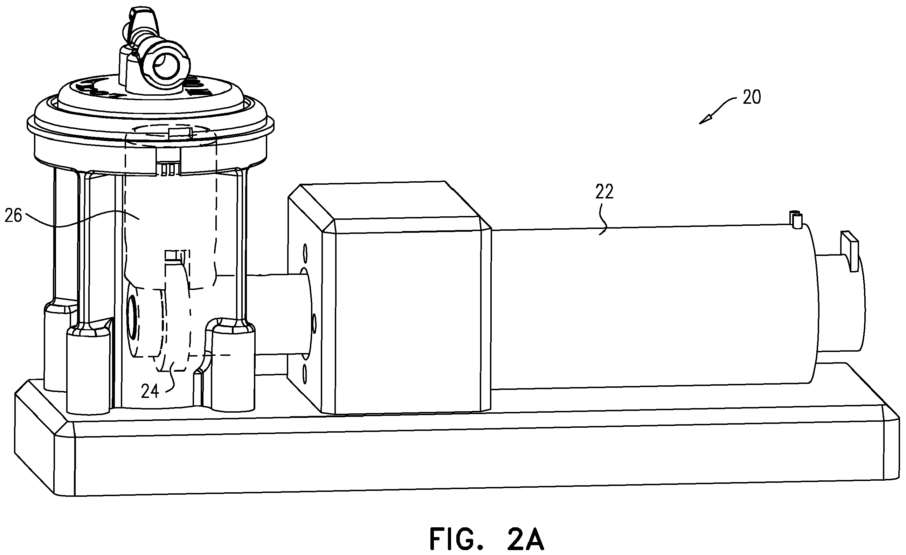

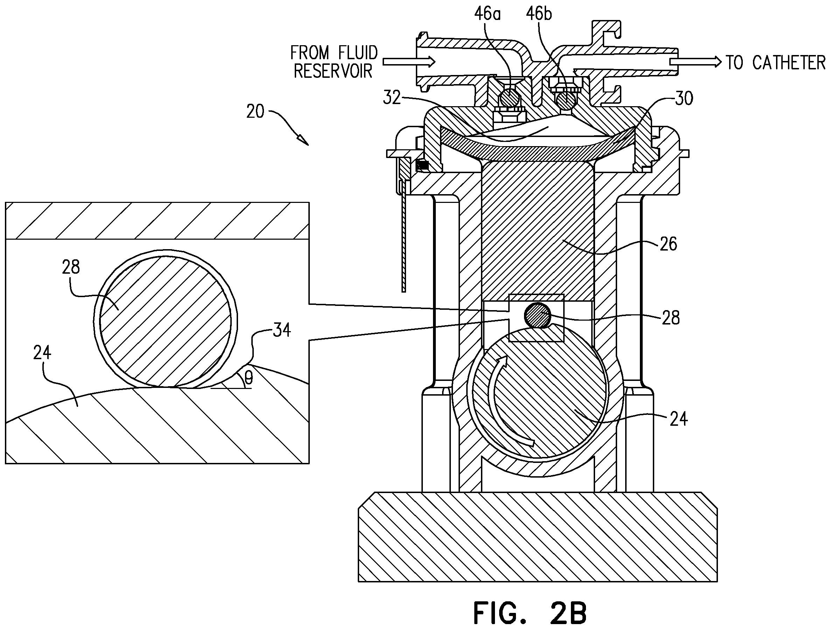

Reference is now made to Figs. 2A-B, which are schematic illustrations of irrigation pump 20, in accordance with some embodiments of the present invention. Fig. 2A shows an isometric view of pump 20, while Fig. 2B shows a cutaway view of a portion of the pump.

Pump 20 comprises a motor 22, which, by turning a snail cam 24, drives a piston 26. Piston 26 is coupled to a diaphragm 30, which defines a wall of a fluid chamber 32. (In the particular example shown, diaphragm 30 defines the bottom wall of fluid chamber 32.) An inlet valve 46a controls the flow of fluid into the fluid chamber from the fluid reservoir, while an outlet valve 46b controls the flow of fluid from the fluid chamber to the catheter.

The snail cam translates rotational motion into linear motion of the piston. Thus, as the snail cam rotates, the snail cam alternatingly (i) pumps fluid from the fluid chamber, and (ii) draws fluid into the fluid chamber, by driving the piston to move the diaphragm. In particular, the rotation of the snail cam may cause the piston to alternatingly (i) push the diaphragm, thus opening outlet valve 46b, closing inlet valve 46a, and forcing fluid out of the fluid chamber and to the catheter, and (ii) pull the diaphragm, thus opening inlet valve 46a, closing outlet valve 46b, and drawing fluid from the fluid reservoir and into the fluid chamber.

In the context of the present application, including the claims, a "snail cam" is any cam that is shaped to define one or more teeth 34 that create the effect of a rapid reversal in the direction of movement of the piston. For example, in Fig. 2B, snail cam 24 is shaped to define a single tooth 34. Proceeding counterclockwise along the circumference the snail cam from the base of tooth 34, the radius of the snail cam increases, e.g., at a constant rate (measured, for example, in mm/radian, or in %/radian), until the apex of the tooth. Upon passing the apex, the radius rapidly decreases, i.e., the radius decreases at a rate that is much greater than the prior rate of increase of the radius. Thus, as the snail cam revolves in the clockwise direction as shown in the figure, the snail cam advances the piston gradually toward the fluid chamber, until the piston rapidly withdraws from the fluid chamber as the piston "drops off" the apex of the tooth.

Since the time during which the piston withdraws is much less than the time during which the piston is advanced, snail cam 24 provides an almost uninterrupted pumping of fluid. For example, the fluid may be pumped for a large majority of, e.g., at least 80%, 90%, or 95% of the duration of, each rotational cycle of the snail cam. Furthermore, in embodiments in which the radius of the snail cam increases at a constant rate, the fluid is pumped at a constant rate (measured in units of volume/time, e.g., cubic centimeters (cc) per second), such a constant rate being advantageous in certain applications.

The rate at which the fluid is pumped is determined by the rate at which the radius of the snail cam increases, as well as the size of the cam, the speed at which the cam revolves, and other factors.

The rate at which the fluid is drawn into the fluid chamber is determined, *inter alia,* by the slope theta (θ) of the tooth. In general, the slope of the tooth is set such as to facilitate a rate of flow into the fluid chamber that is relatively high, yet is low enough such that bubbles are not formed in the fluid. For example, in some embodiments, slope theta is between 50 and 80 degrees.

In light of the above, in the context of the present application, including the claims, a "snail cam" may be alternatively or additionally defined in terms of the effect on the flow of fluid that is produced by rotation of the snail cam. In particular, it may be said that a snail cam is a cam which, when rotating through a rotational cycle at a constant speed of revolution (measured, for example, in radians/second), causes the time during which fluid is pumped from the fluid chamber to be (much) greater than the time during which fluid is drawn into the fluid chamber, e.g., per a ratio of at least 4:1. (This implies that the rate at which fluid is pumped from the fluid chamber is much less than the rate at which fluid is drawn into the fluid chamber.)

Even in light of the alternate definition above, it is noted that, in practice, the snail cam does not necessarily rotate at a constant speed.

As shown in Fig. 2B, in some embodiments, snail cam 24 controls the piston via a peg 28 that is inserted into, and protrudes from, the piston. The snail cam remains in contact with peg 28 for most, or all, of the time during which the snail cam revolves. As the snail cam pushes against the peg, the piston is advanced, and the fluid is forced from fluid chamber 32. Conversely, as peg 28 drops off the apex of the tooth, the piston withdraws from the fluid chamber, and fluid is drawn into the fluid chamber.

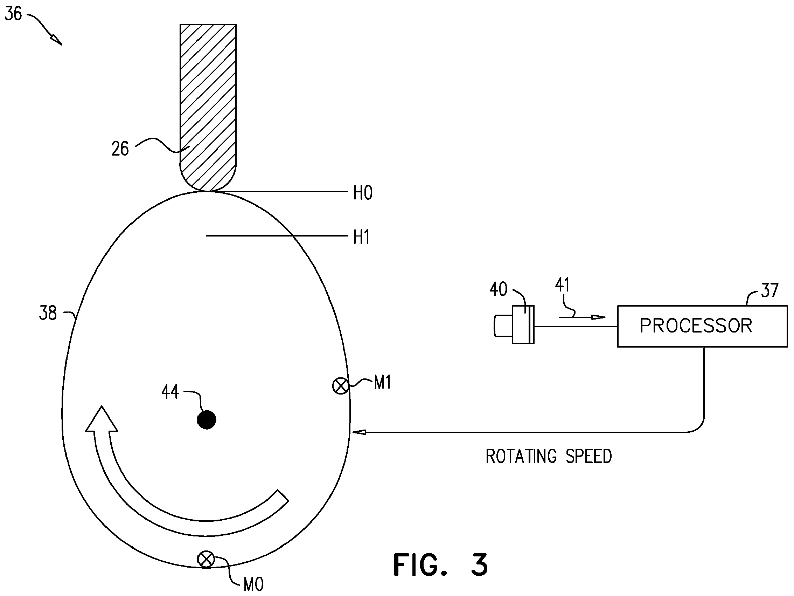

Notwithstanding the particular embodiment shown in Figs. 2A-B, it is noted that the scope of the present invention includes any suitable mechanisms for controlling the flow of fluid via the snail cam and piston. For example, instead of peg 28 resting on the cam, the bottom of the piston may rest directly on the cam, as shown in Fig. 3 (described below), or the piston may be shaped to define a protrusion that rests on the cam. Furthermore, any suitable type of valve may be used to control the inlet and outlet of the fluid chamber. Moreover, snail cam 24 may be shaped to define a plurality of teeth, such that each revolution of the snail cam translates into a plurality of pumping cycles. Although such embodiments increase the number of interruptions in the flow of the fluid, relative to single-tooth embodiments, it may be preferable, in some applications, to have multiple shorter interruptions, rather than a single, longer interruption.

Reference is now made to Fig. 3, which is a schematic illustration of apparatus 36 for pumping fluid, in accordance with some embodiments of the present invention. In general, apparatus 36 may be used in system 21 (Fig. 1) as an alternative to pump 20, which was described hereinabove with reference to Figs. 2A-B.

Apparatus 36 comprises a diaphragm pump, comprising a cam 38, along with the basic pump elements shown in earlier figures, such as piston 26, motor 22, and diaphragm 30. However, apparatus 36 differs from pump 20, at least in that (i) cam 38 is not necessarily a snail cam, and (ii) apparatus 36 further includes a sensor 40, configured to perform the tasks described below. In general, sensor 40 senses the current state of cam 38, and generates a sensor signal 41 in response thereto. Processor 37 (mentioned above with reference to Fig. 1) receives the sensor signal, and controls the rotating speed of the cam (e.g., by controlling motor 22) in response to the state of the cam that is indicated by the sensor signal.

For example, in some embodiments, sensor 40 comprises an optical sensor, which captures images of the cam. The processor receives the images, and then processes the images such as to ascertain the current state of the cam. Such processing may be facilitated by the placement of one or more optical markers on the cam, which are detected, by the processor, in the images.

For example, as shown in the figure, two markers M0 and M1 may be placed on the cam. Upon M0 and M1 being positioned, in the image, as shown, the processor ascertains that the state of the cam is such that the bottom of the piston is at its maximum height H0. Hence, the processor increases the rotating speed of the cam, in order to achieve a rapid drawing of fluid into the fluid chamber.

The cam then rotates at the increased speed, such that the piston quickly drops, and fluid is quickly drawn into the fluid chamber, until the bottom of the piston reaches its minimum height H1. The piston then remains at height H1 until marker M1, which is located at the point at which the distance from the pivot 44 of the cam to the perimeter of the cam again becomes non-constant, comes closest to the piston. Typically the time at H1 is as small as possible, and to achieve this the cam rotates at a third, very quick rate, in this region. The processor then ascertains, based on the positions of M0 and M1, that the state of the cam is such that the piston will momentarily begin to rise. Hence, the processor decreases the rotating speed of the cam, in order to achieve a slow pumping of fluid from the fluid chamber.

It is noted that the embodiment of Fig. 3 is provided by way of example only; in practice, any suitable number of optical markers may be used, these markers being placed on the cam at any suitable locations. In some embodiments, in order to further facilitate the ascertainment of the current state of the cam, the markers may be different from one another, and the processor may be configured to discriminate between the markers.

In all of the embodiments described herein, fluid is drawn into the fluid chamber at a greater rate than that at which fluid is pumped from the fluid chamber. In Figs. 2A-B, the rate differential is achieved by the shape of the cam, while in Fig. 3, the rate differential is achieved by varying the rotating speed of the cam. (In some embodiments, both techniques may be used in combination.) In any case, embodiments of the present invention facilitate nearly uninterrupted pumping of the fluid, e.g., at a constant rate.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### Aspects of the invention:

1. A method, comprising:
   providing (i) a piston, and (ii) a diaphragm coupled to the piston and defining a wall of a fluid chamber; and
   by rotating a cam that drives the piston to move the diaphragm, alternatingly (i) pumping fluid from the fluid chamber, into a body of a subject, at a first rate, and (ii) drawing fluid into the fluid chamber at a second rate that is greater than the first rate.
2. The method according to aspect 1, wherein pumping the fluid comprises pumping the fluid into a heart of the subject.

## Claims

**1.** A pump, comprising:
a piston;
a diaphragm coupled to the piston and defining a wall of a fluid chamber; and
a snail cam configured to, by rotating, alternatingly (i) pump fluid from the fluid chamber, and (ii) draw fluid into the fluid chamber, by driving the piston to move the diaphragm.

**2.** The pump according to claim 1, wherein the snail cam is configured to, when rotating at a constant speed, alternatingly (i) pump fluid from the fluid chamber at a first rate, and (ii) draw fluid into the fluid chamber at a second rate that is greater than the first rate.

**3.** The pump according to claim 1, wherein the snail cam is configured to, when rotating at a constant speed, during each rotational cycle of the snail cam, (i) pump fluid from the fluid chamber for a first amount of time, and (ii) draw fluid into the fluid chamber for a second amount of time that is less than the first amount of time.

**4.** The pump according to claim 3, wherein a ratio of the first amount of time to the second amount of time is at least 4:1.

**5.** The pump according to claim 1, wherein the snail cam is shaped to define a tooth having a slope of between 50 and 80 degrees.

**6.** The pump according to claim 1, wherein the snail cam is shaped to define a plurality of teeth.

**7.** The pump according to claim 1, wherein, over a majority of a circumference of the snail cam, a rate of increase of a radius of the snail cam is constant.

**8.** Apparatus, comprising:
a piston;
a diaphragm coupled to the piston and defining a wall of a fluid chamber;
a cam, configured to, by rotating, alternatingly (i) pump fluid from the fluid chamber, and (ii) draw fluid into the fluid chamber, by driving the piston to move the diaphragm;
a sensor, configured to sense a state of the cam and generate a sensor signal in response thereto; and
a processor, configured to control a rotating speed of the cam in response to the sensor signal.

**9.** The apparatus according to claim 8, wherein the sensor comprises an optical sensor configured to capture an image of the cam, the sensor signal including the image.

**10.** The apparatus according to claim 9, further comprising a plurality of optical markers on the cam, the processor being configured to control the rotating speed of the cam in response to detecting the optical markers in the image.

**11.** The apparatus according to claim 8, wherein the processor is configured to control the rotating speed of the cam such that the cam alternatingly (i) pumps fluid from the fluid chamber at a first rate, and (ii) draws fluid into the fluid chamber at a second rate that is greater than the first rate.

**12.** A method, comprising:
providing (i) a piston, and (ii) a diaphragm coupled to the piston and defining a wall of a fluid chamber; and
by rotating a cam that drives the piston to move the diaphragm, alternatingly (i) pumping fluid from the fluid chamber, into a body of a subject, at a first rate, and (ii) drawing fluid into the fluid chamber at a second rate that is greater than the first rate.

**13.** The method according to claim 12, wherein pumping the fluid from the fluid chamber comprises pumping the fluid for at least 80% of a duration of each rotational cycle of the cam.

**14.** The method according to claim 12, wherein pumping the fluid comprises pumping the fluid at a constant rate.

**16.** The method according to claim 12, wherein the cam is a snail cam.

**16.** The method according to claim 12, comprising controlling a rotating speed of the cam such that the second rate is greater than the first rate.

**17.** The method according to claim 16, wherein controlling the rotating speed of the cam comprises:
using a sensor, sensing a state of the cam and generating a sensor signal in response thereto; and
using a processor, controlling the rotating speed of the cam in response to the sensor signal.

**18.** The method according to claim 17, wherein the sensor includes an optical sensor, and the sensor signal includes an image captured by the optical sensor.

**19.** The method according to claim 18, wherein controlling the rotating speed of the cam comprises controlling the rotating speed of the cam in response to detecting, in the image, optical markers on the cam.
